# EUROPEAN PATENT APPLICATION

(11) **EP 2 386 302 A1**
(43) Date of publication of application: **16.11.2011**
(21) Application number: 11003857.7
(22) Date of filing: 11.05.2011
(51) Int. Cl.: A61K 31/495, A61K 9/28

(54) **A controlled release pharmaceutical dosage form of trimetazidine and processes for the preparation thereof**

(30) Priority: 11.05.2010 IN DE11022010
(71) Applicant: Ranbaxy Laboratories Limited, Delhi 110019 (IN)
(72) Inventor: Bhavarisetti, Murali Krishna, 521185 Krishna, Andhra Pradesh (IN); Kumar, Varinder, 148001 Sangrur, Punjab (IN); Barabde, Umesh Vinayakrao, 444606 Amravati, Maharashtra (IN); Singh, Romi Barat, 221002 Varanasi, Uttar Pradesh (IN)
(74) Representative: Cronin, Brian Harold John

(57) **Abstract**

A controlled release pharmaceutical dosage forms of trimetazidine and the process of preparation thereof, suitable for once-daily dosing is disclosed. The controlled release pharmaceutical dosage form of trimetazidine comprises trimetazidine or its pharmaceutically acceptable salts; one or more gel-forming materials; and one or more gas generating materials.

## Description

### Field of the Invention

The present invention relates to a controlled release pharmaceutical dosage form of trimetazidine suitable for once daily dosing and a process for the preparation thereof..

### Background of the Invention

Trimetazidine, 1-(2,3,4-trimethoxybenzyl piperazine) is a 3-ketoacyl-coenzyme A thiolase inhibitor with a cytoprotective effect, which through the preservation of energy of a cell exposed to the hypoxia or ischaemia, helps avoid the collapse of the intracellular adenosine triphosphate (ATP). Thus ensuring the continued functioning of the ion pumps, and the sodium-potassium transmembrane flux.

Trimetazidine dihydrochloride is used therapeutically in the long-term treatment of angina pectoris. It is freely soluble in water and has two pKa values of 4.32 and 8.95. Trimetazidine dihydrochloride is administered orally in doses of 40 to 60mg in divided doses daily as an immediate release formulation. It is quickly absorbed and eliminated with a plasma half-life of around 6.0 +/- 1.4 hours and Tmax of around 1.8 +/- 0.7 hours. Due to this shorter plasma half life, 20mg preparation is generally given twice or three times a day in order to ensure relatively constant plasma levels.

Servier had developed a modified-release dosage form containing 35mg for twice-daily administration, marketed under the brand name "VASTAREL MR". This dosage form was bioequivalent to the 20mg conventional three times a day formulation of trimetazidine dihydrochloride. The modified-release formulation is based on a hydrophilic matrix that utilizes polymers that swell when in contact with gastrointestinal fluids to form gels. This formulation has been covered by Servier in EP 1108424, which discloses a matrix tablet having a prolonged release of trimetazidine. The formulation includes cellulose derivative polymers. The formulation described releases more than 90% of the drug *in-vitro* within a period of 4 hours.

EP 0673649 describes pharmaceutical compositions for the prolonged release of trimetazidine, wherein such prolonged release is accomplished through the use of a reservoir system. The composition is prepared through compression of the active ingredient mixture and excipients, and then coated with an insoluble polymer.

The prior art describes the use of either hydrophilic polymers in a matrix system or insoluble polymer in a reservoir system for controlling the release of trimetazidine from the pharmaceutical compositions. However, for water-soluble salts, such as, the hydrochloride salt of trimetazidine, it may result in a burst release or dose dump that will result in an increase in side effects.

Controlled release pharmaceutical dosage forms with water-soluble drugs or their salts, wherein the release rate is constant, has always been a challenge to formulators. While various trimetazidine compositions are available commercially, there still exists a need an alternative controlled release pharmaceutical dosage forms that provides a constant release rate for the long term treatment of angina.

Our scientists have now discovered a new gastro-retentive controlled release pharmaceutical dosage form of trimetazidine and processes for the preparation thereof, which is suitable for once daily administration. This formulation exhibits lower incidences of side effects and an increased duration of efficacy.

### Summary of the Invention

In one general aspect, the present invention provides for a controlled release pharmaceutical dosage form of trimetazidine which includes;
(i) trimetazidine or its pharmaceutically acceptable salts;
(ii) one or more gel-forming materials; and
(iii) one or more gas generating materials.

Embodiments of this aspect may include one or more of the following features. For example, the controlled release dosage form has *in vitro* trimetazidine release in USP I apparatus at 100 rpm, in 500 ml 0.1N hydrochloric acid or in 500 ml of pH 6.8 phosphate buffer as follows:
(i) between 0 and 40% after 2 hours;
(ii) between 40 and 60% after 4 hours;
(iii) between 50 and 75% after 6 hours;
(iv) between 60 and 85% after 8 hours;
(v) between 70 and 95% after 12 hours; and
(vi) more than 80% trimetazidine released after 20 hours.

The gel forming material is selected from the group of polyethylene oxide, poloxamers, guar gum, locust bean gum, xanthan gum, cyclodextrin, arabic gum, gellan gum, karaya gum, alginic acid, pectic acid, casein, tara gum, tamarind gum, tragacanth gum, pectin, glucomannan, ghatti gum, arabino galactan, furcelleran, pullulan, carrageenan, alginic acid, glucosamine, chitosan, colloidal silica, pregelatinized starch, hydroxypropyl methyl cellulose, hydroxy propyl cellulose, sodium carboxymethyl cellulose, calcium carboxymethyl cellulose, polyvinyl alcohol, derivatives or combinations thereof. For example, the gel forming material is xanthan gum and/or polyethylene oxide.

The gas generating material is selected from the group of calcium carbonate, sodium glycine carbonate, sodium hydrogen carbonate, potassium hydrogen carbonate, sodium sulfite, sodium bisulfite, sodium metabisulfite, citric acid or its salts such as sodium citrate or calcium citrate, malic acid, tartaric acid, succinic acid, glycolic acid, fumaric acid, maleic acid, ascorbic acid or its salts such as sodium or calcium ascorbate, glycine, sarcosine, alanine, taurine, glutamic acid or combinations thereof. For example, the gas generating material is sodium bicarbonate and/or citric acid.

The controlled release pharmaceutical dosage form may further include one or more pharmaceutically inert excipients. The pharmaceutical dosage form may be in the form of a tablet, capsule, granule, pellet, spheroid, pills or sachet.

In another general aspect, the present invention provides for a process for preparing a controlled release pharmaceutical dosage form, wherein the process includes the steps of:
(i) blending trimetazidine or its pharmaceutically acceptable salts, one or more gel-forming materials, and one or more gas generating materials with one or more pharmaceutically inert excipients;
(ii) optionally granulating the blend of step (i); and
(iii) compressing the blend/granules of steps (i) or (ii) into tablets using appropriate tooling.

In another general aspect, the present invention provides for a process for preparing a controlled release pharmaceutical dosage form wherein the process includes the steps of:
(i) blending trimetazidine or its pharmaceutically acceptable salts, one or more gel-forming materials, one or more gas generating materials with one or more pharmaceutically inert excipients;
(ii) optionally granulating the blend of step (i); and
(iii) filling the blend/granules of steps (i) or (ii) into capsules of suitable size.

In yet another general aspect, the present invention provides for a method of treating angina in mammals, which includes administering to a mammal in need thereof, a controlled release pharmaceutical dosage form of trimetazidine which includes:
(i) trimetazidine or its pharmaceutically acceptable salts;
(ii) one or more gel-forming materials; and
(iii) one or more gas generating materials.

### Detailed Description of the Invention

The term "controlled release" as used herein, includes prolonged release, sustained release, modified release and extended release time frames.

The term "trimetazidine" as used herein includes trimetazidine as well as pharmaceutically acceptable salts, enantiomers, hydrates, polymorphs, solvates, metabolites, prodrugs or mixtures thereof, in particularly trimetazidine dihydrochloride salt. The controlled release pharmaceutical dosage forms of trimetazidine contain less than about 100mg of trimetazidine, in particular, from about 50 mg to about 80 mg of trimetazidine. In one embodiment about 70 mg of trimetazidine is present.

The controlled release pharmaceutical dosage forms of the present invention comprise from about 5% to about 50% of trimetazidine, in particular from about 10% to about 30%, by weight of trimetazidine, based upon the total weight of the dosage form. For example, in one embodiment there is from about 15% to about 25% of trimetazidine.

In one of the object, the present invention relates to controlled release pharmaceutical dosage of the trimetazidine having the desired *in-vitro* and *in-vivo* release profiles. The pharmaceutical dosage form of the present invention has a constant release for up to 24 hours, and thus is suitable for once-a-day administration, making it more patient complaint. It further overcomes the problem of dose-dumping due to burst release of the drug as mentioned in the prior arts. The controlled release pharmaceutical dosage form of the present invention has an *in vitro* trimetazidine release in USP I apparatus at 100 rpm, in 500 ml 0.1N hydrochloric acid or in 500 ml of pH 6.8 phosphate buffer as follows:
(i) between 0 and 40% after 2 hours;
(ii) between 40 and 60% after 4 hours;
(iii) between 50 and 75% after 6 hours;
(iv) between 60 and 85% after 8 hours;
(v) between 70 and 95% after 12 hours; and
(vi) more than 80% trimetazidine released after 20 hours.

The term "gel-forming material", as used herein, forms a stable structure that entraps the generated gas. With the passage of time, the gel-forming material results in a hydrodynamically balanced system whereby the matrix is retained in the stomach for an extended period of time. Simultaneously, the gel-forming polymer provides a slow diffusion pathway for the drug, thereby resulting in a controlled drug release. Specific examples of gel-forming materials include polyethylene oxide, poloxamers, guar gum, locust bean gum, xanthan gum, cyclodextrin, arabic gum, gellan gum, karaya gum, alginic acid, pectic acid, casein, tara gum, tamarind gum, tragacanth gum, pectin, glucomannan, ghatti gum, arabino galactan, furcelleran, pullulan, carrageenan, alginic acid, glucosamine, chitosan, colloidal silica, pregelatinized starch, hydroxypropyl methyl cellulose, hydroxy propyl cellulose, sodium carboxymethyl cellulose, calcium carboxymethyl cellulose, polyvinyl alcohol, derivatives thereof, and the like. These materials can be used alone or in any combination, in particular xanthan gum and/or polyethylene oxide. The controlled release pharmaceutical dosage forms of the present invention comprise from about 5% to about 50%, and in particular from about 10% to about 40%, by weight of the gel-forming material based upon the total weight of the dosage form. For example, in one embodiment the gel-forming material is present from about 25% to 35% by weight based on the total dosage form.

The term "xanthan gum" as used herein, is a high molecular weight biosynthetic polysaccharide gum produced by a pure-culture aerobic fermentation of a carbohydrate with *Xanthomonas campestris.* It is also known as corn sugar gum and is very enzymatically resistant.

The term "polyethylene oxide", as used herein, is a non-ionic homopolymer of the formula -(-O-CH₂-CH₂- )ₙ-, wherein n represents the average number of oxyethylene groups, n generally being from about 2,000 to about 100,000. It is a water soluble resin which is available as a white powder in several grades having different molecular weights which vary in viscosity profile when dissolved in water. Polyethylene oxide is commercially available under the trade name Polyox™ from the Union Carbide Corporation. Commercially available polyethylene oxide products have an average molecular weight of about 100,000 to about 10,000,000. Polyethylene oxide used in the present invention comprises the molecular weight ranging from about 100,000 to about 7000,000.

Since the polyethylene oxide and xanthan gum have complimentary physicochemical properties, control of the release patterns of trimetazidine can be altered by changing the ratio of two polymers present in the formulation. For example, polyethylene oxide is preferentially used for the purpose of increasing viscosity rather than the solubilization of trimetazidine, and xanthan gum is preferentially used for the purpose of forming gels, together with the solubilizing of trimetazidine. The weight ratio between polyethylene oxide and xanthan gum may be in the range of 1:0.1 to 1:10, and in particular 1:1 to 1:10.

The term "gas generating material", as used herein, may be either a single substance or a gas generating couple known to produce gas upon contact with gastric fluid. The gas generating material interacts with water or simply with the gastric fluid to generate carbon dioxide or sulfur dioxide that gets entrapped within the hydrated gel matrix of the gel-forming composition. Specific examples of gas generating materials include, carbonates, such as, calcium carbonate or sodium glycine carbonate; bicarbonates, such as, sodium hydrogen carbonate or potassium hydrogen carbonate; sulfites, such as, sodium sulfite, sodium bisulfite, or sodium metabisulfite.

These salts can be used alone or in combination with an acid source as a couple. The acid source may be one or more of an edible organic acid, a salt of an edible organic acid, or mixtures thereof. Specific examples of organic acids include citric acid or its salts, such as, sodium citrate or calcium citrate; malic acid, tartaric acid, succinic acid, glycolic acid, fumaric acid, maleic acid, or their salts; ascorbic acid or its salts, such as, sodium or calcium ascorbate; glycine, sarcosine, alanine, taurine, glutamic acid. For example, sodium bicarbonate and/or citric acid may be used. Although hydrochloric acid is present in gastric juice, it is included for patients with subacidity. The content of the acidic material can be controlled in such a manner that the final matrix tablet is naturally foamed in purified water and floats. The controlled release pharmaceutical dosage forms of the present invention comprises from about 0% to about 50%, from about 5% to about 40% by weight, and in particular from about 10% to about 25% by weight of the gas generating material based upon the total weight of the dosage form.

Further since the gel containing a polyalkylene oxide is highly elastic, it easily retains carbon dioxide generated from the tablet of the present invention. Accordingly, the density of the matrix is lowered in a few minutes to allow for rapid floating of the preparation. When the gel is rapidly formed and foamed at the same time, the desired controlled release can be achieved in the floating state. If the foaming mechanism is too fast, the shape of the matrix may collapse before the formation of the gel. Alternatively, if the foaming mechanism is too slow, the gel may adhere to the gastric mucosa and any subsequent foaming of the gel will fail to cause floating. This problem can be easily solved by the tablet of the present invention in which the gas generating material and the gel-forming materials containing a polyalkylene oxide are homogeneously dispersed.

The term "pharmaceutical dosage form", as used herein, includes any conventional dosage form, such as, tablets, capsules, granules, pellets, spheroids, pills, sachets and the like.

The controlled release pharmaceutical dosage form of the present invention may further comprise one or more pharmaceutically inert excipients selected from diluents, binders, lubricant/glidants and coloring agents.

Specific examples of diluents include dicalcium phosphate, dihydrogen calcium phosphate, tribasic calcium phosphate, calcium carbonate, calcium sulphate, lactose, microcrystalline cellulose, kaolin, pregelatinized starch, and the like, or combinations thereof.

Specific examples of binders include polyvinylpyrrollidone, gelatin, polyvinyl alcohol, gum acacia, or combinations thereof. Part or whole of the binder may be present in the intragranular portion or may be added in the binder solution for granulation.

Specific examples of granulating fluid include acetone, ethanol, isopropyl alcohol, methylene chloride or combinations thereof.

Specific examples of lubricants/glidants include colloidal silicon dioxide, stearic acid, magnesium stearate, calcium stearate, zinc stearate, sodium stearyl fumarate, talc, hydrogenated castor oil, sucrose esters of fatty acid, microcrystalline wax, yellow beeswax, white beeswax, or combinations thereof.

Coloring agents include any FDA approved color for oral use.

The controlled release pharmaceutical dosage form may be prepared by the conventional techniques known in the art, such as, wet granulation, dry granulation, direct compression or extrusion-spheronization or hot melt extrusion. The wet granulation process involves the use of water or any other suitable granulating fluid. The dry granulation may involve the use of a roller compacter or any suitable technique.

The controlled release pharmaceutical dosage form of the present invention may be further coated with one or more non-functional coating layers, which may include film forming polymers with/without coating additives.

Coating additives may be selected from plasticizers, opacifiers, coloring agents, lubricants/glidants, and the like.

Examples of film-forming polymers include ethylcellulose, hydroxypropyl methylcellulose, hydroxypropylcellulose, methylcellulose, carboxymethyl cellulose, hydroxymethylcellulose, hydroxyethylcellulose, cellulose acetate, hydroxypropyl methylcellulose phthalate, cellulose acetate phthalate, cellulose acetate trimellitate; waxes, such as, polyethylene glycol; methacrylic acid polymers, such as, Eudragit®; and the like. Alternatively, commercially available coating compositions comprising film- forming polymers marketed under various trade names, such as Opadry® may also be used.

Specific examples of plasticizers include triethylcitrate, dibutylsebacate, acetylated triacetin, tributylcitrate, glyceroltributyrate, monoglyceride, rape oil, olive oil, sesame oil, acetyltributylcitrate, acetyltriethylcitrate, glycerin sorbitol, diethyloxalate, diethyl phthalate, diethylmalate, diethylfumarate, dibutylsuccinate, diethylmalonate, dioctylphthalate, or combinations thereof.

Specific examples of opacifiers include titanium dioxide, manganese dioxide, iron oxide, silicon dioxide, or combinations thereof.

Coating may be performed by applying the coating composition as a solution/suspension/blend using any conventional coating technique known in the prior art, such as, spray coating in a conventional coating pan or fluidized bed processor; dip coating or compression coating.

Examples of solvents used for preparing solution/dispersion of coating substances include methylene chloride, isopropyl alcohol, acetone, methanol, ethanol, water and the like.

In one of the embodiment, a controlled release pharmaceutical dosage form of trimetazidine may be prepared by a process, which includes the steps of:
(i) blending trimetazidine or its pharmaceutically acceptable salts, one or more gel-forming materials, and one or more gas generating materials with one or more pharmaceutically inert excipients;
(ii) granulating the blend of step (i) with a binder solution;
(iii) compressing the granules of step (ii) into tablets using appropriate tooling; and
(iv) optionally coating the tablets of step (iii) with an Opadry® dispersion.

In another embodiment, a controlled release pharmaceutical dosage form of trimetazidine may be prepared by a process, which includes the steps of:
(i) blending trimetazidine or its pharmaceutically acceptable salts, one or more gel-forming materials, and one or more gas generating materials with one or more pharmaceutically inert excipients;
(ii) compressing the blend of step (i) into tablets using appropriate tooling; and
(iii) optionally coating the tablets of step (ii) with Opadry® dispersion.

In another embodiment, a controlled release pharmaceutical dosage form of trimetazidine may be prepared by a process which includes the steps of:
(i) blending trimetazidine or its pharmaceutically acceptable salts, one or more gel-forming materials, and one or more gas generating materials with one or more pharmaceutically inert excipients,
(ii) optionally granulating the blend of step (i),
(iii) filling the blend/granules of steps (i) or (ii) into capsules of a suitable size.

The present invention also provides for a method of treating angina in mammals. The method includes administering to a mammal in need thereof, the controlled release pharmaceutical dosage form of trimetazidine. The controlled release pharmaceutical dosage form of the present invention may be administered in combination with one or more additional other cardio therapeutic agents.

The invention may be further illustrated by the following examples, which are for illustrative purpose only and should not be construed as limiting the scope of the invention in any way.

### EXAMPLE 1

| **S. No.** | **Ingredients** | **% w/w based on the total dosage form** |
|---|---|---|
| **Intragranular** | | |
| 1. | Trimetazidine Dihydrochloride | 17.07 |
| 2. | Xanthan gum | 29.27 |
| 3. | Microcrystalline Cellulose (Avicel-PH 112) | 12.19 |
| 4. | Lactose Anhydrous (DCL21) | 6.10 |
| 5. | Sodium Bicarbonate | 14.63 |
| 6. | Citric Acid (anhydrous) | 7.32 |
| **Binder Solution** | | |
| 7. | Polyvinylpyrrolidone (PVP-K30) | 6.58 |
| 8. | Isopropyl Alcohol | q.s. |
| **Extragranular** | | |
| 9. | Colloidal silicon dioxide (Aerosil 200)/Talc | 1.22 |
| 10. | Magnesium Stearate | 1.22 |
| **Tablet weight of the core tablet** | | **392.00** |
| **Coating** | | |
| 11. | Opadry OY-S-58910 | 4.39 |
| 12. | Purified water | q.s. |
| **Total weight of the tablet** | | **410.00** |

### EXAMPLE 2

| **S. No.** | **Ingredients** | **% w/w based on the total dosage form** |
|---|---|---|
| **Intragranular** | | |
| 1. | Trimetazidine Dihydrochloride | 20.71 |
| 2. | Xanthan gum | 29.58 |
| 3. | Microcrystalline Cellulose (Avicel-PH 112) | 11.83 |
| 4. | Lactose Anhydrous (DCL21) | 7.39 |
| 5. | Polyvinylpyrrolidone (PVP-K30) | 4.44 |
| 6. | Sodium Bicarbonate | 8.87 |
| 7. | Citric Acid (anhydrous) | 4.44 |
| **Binder Solution** | | |
| 8. | Polyvinylpyrrolidone (PVP-K30) | 4.44 |
| 9. | Isopropyl Alcohol | q.s. |
| **Extragranular** | | |
| 9. | colloidal silicon dioxide (Aerosil 200)/Talc | 1.48 |
| 10. | Magnesium Stearate | 1.48 |
| **Tablet weight of the core tablet** | | **320.00** |
| **Coating** | | |
| 11. | Opadry OY-S-58910 | 5.32 |
| 12. | Purified water | q.s. |
| **Total weight of the tablet** | | **338.00** |

### Procedure of Examples 1 and 2:

1. Ingredients 1-6 (ingredients 1-7 of example 2) were passed through suitable sieve and transferred into a rapid mixer granulator and mixed.
2. Polyvinylpyrrolidone was dissolved in isopropyl alcohol under continuous stirring.
3. The premix of step 1 was granulated using the binder solution of step 2.
4. The wet mass of step 3 was dried in a suitable dryer, milled using a suitable screen and transferred into a blender.
5. Ingredients 9-10 (ingredients 10-11 of example 2) were sifted through a suitable sieve and transferred into the step 4 blender and blended.
6. The lubricated blend of step 5 was compressed using suitable punches.
7. Opadry® was suspended in purified water under continuous stirring.
8. The tablets of step 6 were coated using step 7 coating dispersion.

### EXAMPLE 3

| **S. No.** | **Ingredients** | **% w/w based on the total dosage form** |
|---|---|---|
| **Core** | | |
| 1. | Trimetazidine Dihydrochloride | 21.34 |
| 2. | Xanthan gum | 30.49 |
| 3. | Microcrystalline Cellulose (Avicel-PH 112) | 12.19 |
| 4. | Lactose Anhydrous (DCL21) | 7.62 |
| 5. | Sodium Bicarbonate | 9.15 |
| 6. | Citric Acid (anhydrous) | 4.57 |
| 7. | Polyvinylpyrrolidone (PVP-K30) | 8.23 |
| 8. | Colloidal silicon dioxide (Aerosil 200)/Talc | 1.22 |
| 9. | Magnesium Stearate | 1.22 |
| **Tablet weight of the core tablet** | | **315.00** |
| **Coating** | | |
| 10. | Opadry OY-S-58910 | 3.96 |
| 11. | Purified water | q.s. |
| **Total weight of the tablet** | | **328.00** |

### Procedure of Example 3:

1. Trimetazidine dihydrochloride was passed along with xanthan gum through a suitable sieve.
2. The ingredients 3-7 were passed through a suitable sieve and all the sifted ingredients were transferred into the blender, and mixed for suitable duration.
3. Ingredients 8-9 were passed through a suitable sieve and transferred into the blend of step 3 and blended for a suitable duration.
4. The blend of step 3 was compressed into tablets using suitable punches.
5. Opadry® was suspended in purified water under continuous stirring.
6. Tablets of step 4 were coated using step 5 coating dispersion.

### In-vitro Dissolution study

*In-vitro* trimetazidine release from the tablet prepared as per the pharmaceutical composition of the examples 1 and 2 were determined by dissolution of trimetazidine using the USP I apparatus at 100 rpm, in 500 ml 0.1N hydrochloric acid for 20 hours. The results of the release studies are represented below in the Table 1.

**Table 1: In-vitro Trimetazidine release (USP I apparatus at 100 rpm, in 500 ml 0.1N hydrochloric acid)**

| **Time (hours)** | **Cumulative *in-vitro* trimetazidine release (%w/w)** | |
|---|---|---|
| | **Example 1** | **Example 2** |
| 0 | 0 | 0 |
| 1 | 21 | 22 |
| 2 | 32 | 36 |
| 3 | 41 | 47 |
| 4 | 48 | 56 |
| 6 | 65 | 70 |
| 8 | 78 | 79 |
| 12 | 91 | 88 |
| 16 | 96 | 91 |
| 20 | 99 | 93 |

*In-vitro* trimetazidine release from the tablet prepared as per the pharmaceutical composition of the examples 1-3 was determined by the dissolution of trimetazidine using the USP I apparatus at 100 rpm, in 500 ml of phosphate buffer of pH 6.8 for 20 hours. The results of the release studies are represented below in the Table 2.

**Table 2: In-vitro Trimetazidine release (USP I apparatus at 100 rpm, in 500 ml of phosphate buffer of pH 6.8)**

| **Time (hours)** | **Cumulative *in-vitro* trimetazidine release (%w/w)** | | |
|---|---|---|---|
| | **Example 1** | **Example 2** | **Example 3** |
| 0 | 0 | 0 | 0 |
| 1 | 20 | 20 | 20 |
| 2 | 31 | 32 | 32 |
| 3 | 39 | 42 | 41 |
| 4 | 46 | 51 | 51 |
| 6 | 56 | 64 | 65 |
| 8 | 65 | 74 | 74 |
| 12 | 78 | 87 | 85 |
| 16 | 86 | 94 | 91 |
| 20 | 90 | 97 | 95 |

While there has been shown and described what are the preferred embodiments of the invention, one; skilled in the pharmaceutical formulation art will appreciate that various modifications in the dosage forms and process can be made without departing from the scope of the invention as it is defined by the appended claims.

## Claims

1. A controlled release pharmaceutical dosage form of trimetazidine comprising;
(i) trimetazidine or its pharmaceutically acceptable salts;
(ii) one or more gel-forming materials; and
(iii) one or more gas generating materials.

2. The controlled release pharmaceutical dosage form of claim 1, wherein the dosage form has *in vitro* trimetazidine release in USP I apparatus at 100 rpm, in 500 ml 0.1N hydrochloric acid or in 500 ml of pH 6.8 phosphate buffer as follows:
(i) between 0 and 40% after 2 hours;
(ii) between 40 and 60% after 4 hours;
(iii) between 50 and 75% after 6 hours;
(iv) between 60 and 85% after 8 hours;
(v) between 70 and 95% after 12 hours; and
(vi) more than 80% trimetazidine released after 20 hours.

3. The controlled release pharmaceutical dosage form of claim 1, wherein the gel forming material is selected from the group consisting of polyethylene oxide, poloxamers, guar gum, locust bean gum, xanthan gum, cyclodextrin, arabic gum, gellan gum, karaya gum, alginic acid, pectic acid, casein, tara gum, tamarind gum, tragacanth gum, pectin, glucomannan, ghatti gum, arabino galactan, furcelleran, pullulan, carrageenan, alginic acid, glucosamine, chitosan, colloidal silica, pregelatinized starch, hydroxypropyl methyl cellulose, hydroxy propyl cellulose, sodium carboxymethyl cellulose, calcium carboxymethyl cellulose, polyvinyl alcohol, derivatives or combinations thereof.

4. The controlled release pharmaceutical dosage form of claim 3, wherein the gel forming material comprises xanthan gum and/or polyethylene oxide.

5. The controlled release pharmaceutical dosage form of claim 1, wherein the gas generating material is selected from the group consisting of calcium carbonate, sodium glycine carbonate, sodium hydrogen carbonate, potassium hydrogen carbonate, sodium sulfite, sodium bisulfite, sodium metabisulfite, citric acid or its salts such as sodium citrate or calcium citrate, malic acid, tartaric acid, succinic acid, glycolic acid, fumaric acid, maleic acid, ascorbic acid or its salts such as sodium or calcium ascorbate, glycine, sarcosine, alanine, taurine, glutamic acid or combinations thereof.

6. The controlled release pharmaceutical dosage form of claim 5, wherein the gas generating material comprises sodium bicarbonate and/or citric acid.

7. The controlled release pharmaceutical dosage form of any of the preceding claims may further comprise one or more pharmaceutically inert excipients.

8. The controlled release pharmaceutical dosage form of any of the preceding claims wherein the pharmaceutical dosage form may be in the form of a tablet, capsule, granule, pellet, spheroid, pills or sachet.

9. A process for preparing a controlled release pharmaceutical dosage form wherein the process comprises the steps of:
(i) blending trimetazidine or its pharmaceutically acceptable salts, one or more gel-forming materials, and one or more gas generating materials with one or more pharmaceutically inert excipients;
(ii) optionally granulating the blend of step (i); and
(iii) compressing the blend/granules of steps (i) or (ii) into tablets using appropriate tooling.

10. A process for preparing a controlled release pharmaceutical dosage form wherein the process comprises the steps of:
(i) blending trimetazidine or its pharmaceutically acceptable salts, one or more gel-forming materials, one or more gas generating materials with one or more pharmaceutically inert excipients;
(ii) optionally granulating the blend of step (i); and
(iii) filling the blend/granules of steps (i) or (ii) into capsules of suitable size.

11. A method of treating angina in mammals, which comprises administering to a mammal in need thereof, the controlled release pharmaceutical dosage form of trimetazidine which comprises:
(i) trimetazidine or its pharmaceutically acceptable salts;
(ii) one or more gel-forming materials; and
(iii) one or more gas generating materials.
